# EUROPEAN PATENT APPLICATION

(11) **EP 4 721 687 A1**
(43) Date of publication of application: **08.04.2026**
(21) Application number: 25205652.8
(22) Date of filing: 30.09.2025
(51) Int. Cl.: A61B 18/18, A61B 34/10

(54) **METHOD AND APPARATUS IN SUPPORT OF ABLATING A TARGET VOLUME**

(30) Priority: 02.10.2024 US 202418904182
(71) Applicant: Varian Medical Systems, Inc., Palo Alto, CA 94304 (US)
(72) Inventor: FALLAHI, Hojjatollah, Minneapolis, 55401 (US); OLSON, Dane, New Hope, 55427 (US); BOIVIN, Gael Luis, 8032 Zurich (CH)
(74) Representative: Mathisen & Macara LLP

(57) **Abstract**

A control circuit can access (201) three-dimensional image information (202) of a target volume for a particular patient as well as ablation zone information (204) corresponding to a particular ablation treatment context. The control circuit can then register (205) the three-dimensional image information of the target volume with the ablation zone information to generate resultant information representing a radiation treatment plan, and then generate (206) a two-dimensional representation of target volume coverage by a corresponding ablation zone as a function of the resultant information. That two-dimensional representation of target volume coverage by the corresponding ablation zone can then be presented (207) via a user interface.

## Description

### TECHNICAL FIELD

These teachings relate generally to treating a patient's target volume with energy and more particularly to identifying an optimal plan for ablating a patient's target volume.

### BACKGROUND

The use of energy to treat medical conditions comprises a known area of prior art endeavor. For example, microwave ablation is a treatment option for tumors (including solid tumors) in soft tissues such as the liver, kidney, and lungs. This type of treatment is typically performed by an interventional radiologist in an outpatient setting. A microwave ablation needle is inserted into the target location under image guidance, such as computed tomography (CT) or ultrasound. By radiating microwave energy through the needle tip, the temperature of the target tissue is raised to lethal levels. Successful outcomes in tumor destruction and increased survival rates rely in considerable part on treatment planning and needle navigation.

Radiologists sometimes utilize three-dimensional reconstructions of organs from CT scans to identify an optimal entry point for the ablation needle. The objective is often to position the active zone of the needle at the center of the target volume while minimizing damage to surrounding tissues such as the skin, muscles, and other so-called organs-at-risk, and also while avoiding critical structures.

### BRIEF DESCRIPTION OF DRAWINGS

Various needs in the foregoing regards are at least partially met through provision of the method and apparatus in support of ablating a target volume described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a graph as configured in accordance with various embodiments of these teachings;
FIG. 4 comprises a graph as configured in accordance with various embodiments of these teachings;
FIG. 5 comprises a graph as configured in accordance with various embodiments of these teachings;
FIG. 6 comprises a graph as configured in accordance with various embodiments of these teachings;
FIG. 7 comprises a graph as configured in accordance with various embodiments of the invention;
FIG. 8 comprises a graph as configured in accordance with various embodiments of these teachings;
FIG. 9 comprises a graph as configured in accordance with various embodiments of the invention;
FIG. 10 comprises a schematic representation as configured in accordance with various embodiments of these teachings;
FIG. 11 comprises a graph as configured in accordance with various embodiments of the invention;
FIG. 12 comprises a schematic representation as configured in accordance with various embodiments of the invention; and
FIG. 13 comprises a graph as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Ablation systems have been developed that use electromagnetic tracking combined with CT or ultrasound, or laser systems integrated with CT scanners. Once an appropriate ablation needle insertion angle and depth are determined, potential ablation zones created by the selected ablation needle have been overlaid onto the target area. This approach provides the radiologist with a three-dimensional visual representation to help ascertain whether the tumor is adequately covered by the selected ablation zone.

The applicant has determined that such prior art approaches are overly qualitative and unduly subjective. Three-dimensional visualization alone does not offer a quantitative and user-friendly measure to assure that an optimal plan has been identified. Furthermore, the applicant has determined that comparing different treatment scenarios for a given patient by adjusting input treatment parameters, ablation needle insertion depth and angle, and ablation needle repositioning is typically not straightforward. Furthermore, the applicant has determined that the prior art lack of any quantitative measure to assess the quality of an ablation plan makes it difficult or impossible to compare the success rates of different scenarios between practitioners and/or institutions. Shareable metrics that can greatly facilitate the standardization of a given practice, which in turn can improve standard practice, are therefore unfortunately lacking.

Generally speaking, pursuant to these various embodiments, a control circuit accesses three-dimensional image information of a target volume for a particular patient as well as ablation zone information corresponding to a particular ablation treatment context. The control circuit then registers the three-dimensional image information of the target volume with the ablation zone information to generate resultant information representing a treatment plan and then generates a two-dimensional representation of target volume coverage by a corresponding ablation zone as a function of the resultant information. The control circuit can then present the two-dimensional representation of target volume coverage by the corresponding ablation zone via a user interface, such as on a computer display. Following the presentation of the two-dimensional representation of target volume coverage by the corresponding ablation zone, the control circuit determines when a user rejects the radiation treatment plan, and then adjusts the ablation treatment context in response to the user rejection. The process then continues until a user accepts the radiation treatment plan.

By one approach, the aforementioned three-dimensional image information of the target volume can comprise three-dimensional computed tomography imagery.

By one approach, the aforementioned ablation zone information can comprise microwave ablation zone information. In lieu of the foregoing or in combination therewith, the aforementioned ablation zone information can comprise information that is based, at least in part, on an ablation zone dimension chart for a particular ablation needle. These teachings are highly flexible in practice and will accommodate the aforementioned particular ablation treatment context comprising a pre-treatment ablation treatment context or a post-treatment ablation treatment context.

By one approach, the aforementioned two-dimensional representation of the target volume coverage by the corresponding ablation zone can comprise a two-dimensional graph that depicts a percentage of the target volume that is covered by the corresponding ablation zone. If desired, this graph can further depict a percentage of the target volume that is fully ablated by the corresponding ablation zone. And, in lieu of the foregoing or in combination therewith, the graph can depict a percentage of the target volume that is fully ablated by the corresponding ablation zone for each of a gross tumor volume, a clinical tumor volume, and a planning target volume.

By one approach, the aforementioned two-dimensional representation of the target volume coverage by the corresponding ablation zone can be agnostic with respect to both patient and ablation needle specifics to thereby allow for direct comparison with ablation results from other patients and/or ablation needles.

So configured, these teachings can support and facilitate registering a three-dimensional CT scan of a target inside a patient organ to an expected and/or prescribed three-dimensional ablation zone that is reconstructed from the needle manufacturer's ablation zone dimension charts and then generating a two-dimensional representation of target coverage by the ablation zone. This representation can be a plot that shows what percentage of the target is covered/destroyed by the selected ablation zone.

Typical prior art approaches provide only a three-dimensional visual and qualitative representation of ablation zone coverages. The present teachings, however, can provide a two-dimensional representation that is easier to interpret and compare. These teachings will support presenting multiple treatment scenarios on a two-dimensional histogram with regards to standard deviation of ablation zone dimensions or volumes, different ablation input power and/or time, needle insertion depth, and so forth to aid a clinician to identify an optimal ablation treatment plan.

In addition, the applicant has determined that, in practice, a three-dimensional visual and qualitative representation as is typically utilized in the prior art is dependent on the shape, size, and volume of the targeted lesion. Such information is accordingly very patient specific, and that, in turn, makes it difficult to compare results from one patient to another. As a result, a patient cohort of statistical value cannot readily be analyzed in a comprehensive manner and instead would require arbitrary pooling/subclassification of the patients, an approach that can greatly hobble and limit the value of such a study. The present teachings overcome such limitations, at least in substantial part and hence considerably improve ablation planning technologies and techniques.

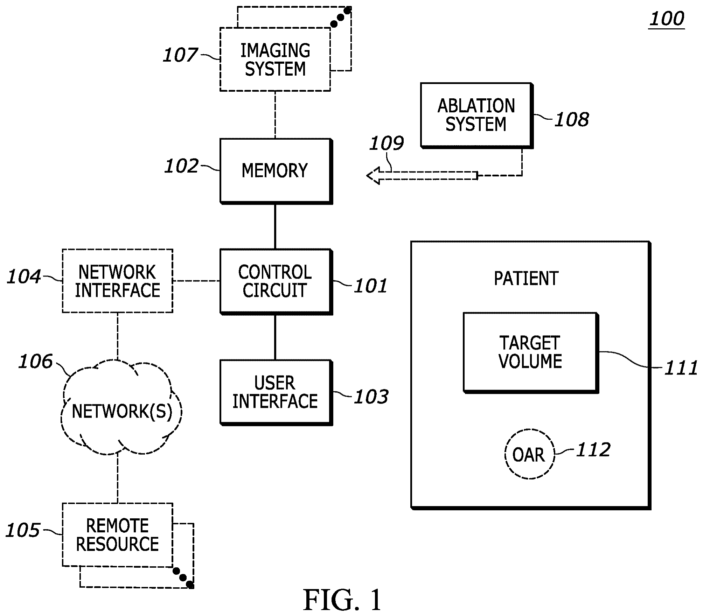

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as three-dimensional image information of a target volume for a particular patient and ablation zone information corresponding to a particular ablation treatment context as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

The control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to one or more network interfaces 104 to thereby provide access to one or more remote resources 105 that are external to the apparatus 100. Numerous examples are known in the art. A non-exhaustive listing would include Universal Serial Bus (USB)-based interfaces, RS232-based interfaces, I.E.E.E. 1394 (aka Firewire)-based interfaces, Ethernet-based interfaces, any of a variety of so-called Wi-Fi^{™}-based wireless interfaces, Bluetooth^{™}-based wireless interfaces, cellular telephony-based wireless interfaces, Near Field Communications (NFC)-based wireless interfaces, standard telephone landline-based interfaces, cable modem-based interfaces, and digital subscriber line (DSL)-based interfaces. Such interfaces can be selectively employed to communicatively couple the control circuit 101 another such machine, to a local area network, or to any of a variety of wide area networks 106 (such as, but not limited to, the Internet). So configured the control circuit 101 can communicate with other elements (both within the apparatus 100 and external thereto) via the network interface.

By one approach, the apparatus operably couples to or otherwise includes one or more imaging systems 107. As one illustrative example in these regards, one such imaging system 107 can comprise a computed tomography system. Such an imaging system can provide, for example, some or all of the three-dimensional image information of a target volume for a particular patient as referenced herein.

This apparatus 100 serves in an application setting that includes an ablation system 108. (Some ablation modalities include microwave ablation, radiofrequency ablation, cryoablation, laser ablation, high-intensity focused ultrasound ablation, and irreversible electroporation, to note but a few.) For the sake of an illustrative example, but without intending to suggest any limitations in these regards, this description will presume the ablation system 108 to be a microwave ablation system. Typical components of a microwave ablation system include a microwave generator, a power supply and control unit, an ablation needle 109 (sometimes also referred to as an ablation probe or antenna), a transmission line that connects the microwave generator to the ablation needle, an imaging guidance system, and possibly a cooling system and/or a thermometry system.

This apparatus 100 also serves, per these teachings, with respect to a particular patient 110 having at least one target volume (such as a tumor) to be ablated and possibly one or more organs-at-risk (OAR) 112 that require consideration as well.

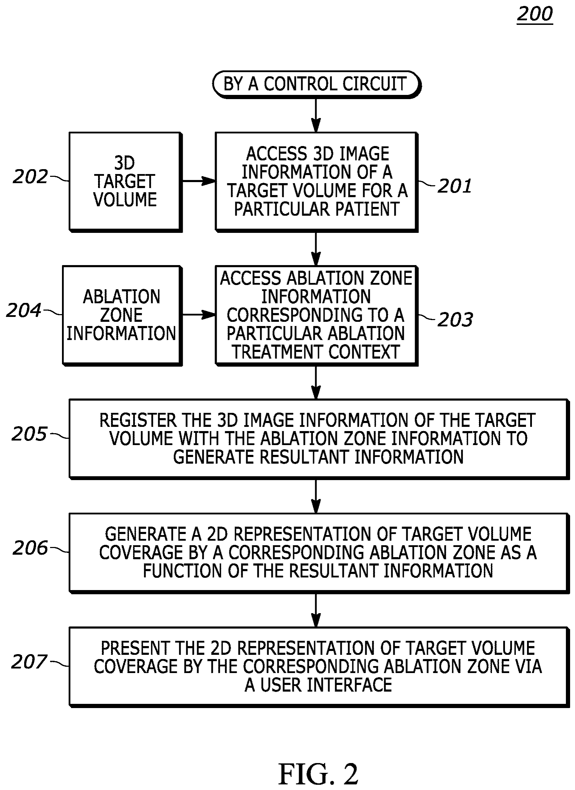

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 by providing the clinician with information that was not heretofore otherwise readily available using existing technology approaches and techniques.

At block 201, this process 200 accesses three-dimensional image information 202 of a target volume 111 for a particular patient 110. By one approach, this three-dimensional image information 202 comprises three-dimensional computed tomography imagery (as sourced, for example, via the aforementioned imaging system 107). Three-dimensional computed tomography imagery is an imaging technique that uses computer-processed combinations of multiple X-ray measurements taken from different angles to produce a three-dimensional image of a specific area of the scanned object.

At block 203, the control circuit 101 accesses ablation zone information 204 that corresponds to a particular ablation treatment context. As used herein, the word "context" refers the specific set of circumstances, conditions, and/or factors in which the ablation treatment is being considered or applied. Accordingly, the context can refer to any or all of medical context (such as the type of medical condition being treated, the patient's overall health, and other treatments that have been tried or are being used concurrently), technical context (such as the specific type of ablation being used (e.g., radiofrequency ablation, cryoablation, laser ablation), as well as the equipment and technical expertise required, the ablation input power, the ablation time, the ablation needle size, the ablation needle insertion depth, the ablation needle insertion angle etc.), procedural context (such as the setting in which the ablation is being performed, such as in an operating room under general anesthesia or in an outpatient clinic with local anesthesia), and/or patient context (such as information that is specific to the individual patient, including their medical history, personal preferences, potential risk factors, and their ability to tolerate the procedure).

In keeping with the current illustrative example, this ablation zone information comprises microwave ablation zone information. Accordingly, the ablation zone information 204 can comprise information that is based, at least in part, on an ablation zone dimension chart for a particular ablation needle 109. An ablation zone dimension chart is a reference tool (typically provided by the manufacturer of the chart) that provides information about the expected size and shape of the tissue ablation zone created by a specific microwave ablation needle/antenna. Examples of the kind of information that may be included in an ablation zone dimension chart are antenna type, power settings (as different power settings can lead to different sizes of ablation zones), ablation time (referring to the duration for which the microwave energy is applied), tissue type (as different tissues can have different electrical and thermal properties that can affect the size of the ablation zone), ablation zone dimensions (that is, the actual dimensions of the ablation zone, which are often provided in terms of diameter and length (and sometimes as a range)), temperature distribution (regarding, for example, the temperature distribution within the ablation zone, indicating, for example, regions of highest temperature and how the temperature may decrease outward from the center), and so forth. Many charts also include graphical representations comprising diagrams and/or images that show the shape of the ablation zone, which may be spherical, ellipsoidal, or cylindrical, depending on the needle design and operating parameters.

These teachings are flexible in practice. Accordingly, these teachings will accommodate a particular ablation treatment context that comprises a pre-treatment ablation treatment context. By another approach, these teachings will also accommodate a particular ablation treatment context that comprises a post-treatment ablation treatment context.

At block 205, the control circuit 101 then registers the three-dimensional image information 202 of the target volume 111 with the ablation zone information 204 to generate corresponding resultant information. The ablation zone information 204 used for this purpose may comprise, for example, image-based information. Image registration is a process used in medical imaging to align two or more images so that they can be compared or combined in a meaningful way. When registering one three-dimensional image of a tumor with another image (or images) that show an ablation zone, an accurate registration can depict the relationship between the tumor and the area to be treated (or that has been treated) by ablation.

At block 206, the control circuit 101 generates a two-dimensional representation of the target volume coverage by a corresponding ablation zone as a function of the aforementioned resultant information, the resultant information representing the radiation treatment plan 113. This two-dimensional representation of the target volume coverage by the corresponding ablation zone can comprise, by one approach, a graph that depicts a percentage of the target volume that is covered by the corresponding ablation zone. The latter may include, if desired, a depiction of a percentage of the target volume that is fully ablated by the corresponding ablation zone.

In a treatment planning context, clinicians often make use of a gross tumor volume (which refers to the volume of the tumor that can be seen or identified using imaging techniques and/or physical examination; the gross tumor volume therefore represents the detectable extent of malignant growth), a clinical tumor volume (which usually includes both the gross tumor volume and additional adjacent areas of potential microscopic disease spread; the clinical tumor volume helps to account for uncertainties with respect to the extent of the tumor), and a planning target volume (which typically comprises an expansion beyond the clinical tumor volume that accounts for various factors that can lead to discrepancies between a planned ablation zone and the zone as actually delivered such as uncertainties in treatment delivery). By one approach, these teachings will accommodate the aforementioned graph depicting a percentage of the target volume that is fully ablated by the corresponding ablation zone for each of a gross tumor volume, a clinical tumor volume, and a planning target volume.

At block 207, the control circuit 101 presents the aforementioned two-dimensional representation of target volume coverage by the corresponding ablation zone via the aforementioned user interface 103. For example, the aforementioned two-dimensional representation of target volume coverage by the corresponding ablation zone may be presented to a user on a computer display.

An optimized radiation treatment plan 113 may then be generated, since a clinician is able to view information that was not heretofore otherwise readily available using existing technology approaches and techniques. The aforementioned two-dimensional representations enable rapid validation/rejection by a clinician of a given treatment plan 113 independently of the original imaging. The aforementioned two-dimensional representations enable a clinician to determine whether the radiation treatment plan 113 provides optimal ablation zone dimensions or volumes and/or optimal ablation input power and/or time.

Typical prior art technological approaches provide a three-dimensional visual and qualitative representation of ablation zone coverages while the present technological teachings provide a two-dimensional representation that is easier to interpret and compare. By one approach, multiple treatment scenarios can be shown on a two-dimensional histogram with regards to standard deviation of ablation zone dimensions or volumes, different ablation input power and/or time, needle insertion depth, and so forth. In addition, the evaluation of a three-dimensional representation can be time-consuming and prone to interpretive error. More particularly, to fully evaluate the quality of a plan with current techniques, clinicians must observe and work through the complete tumor volume and relative CT-scan slides. In contrast, the two-dimensional representation offered by the present teachings can enable rapid validation/rejection of a given plan independently of the original imaging.

Accordingly, a clinician is able to consider the two-dimensional histograms presented at the user interface 103 and either accept or reject a given plan. If the clinician rejects a given plan, the process returns to block 203, and one or more ablation treatment contexts (such as ablation input power; ablation time; ablation needle size; ablation needle type; ablation needle insertion depth; ablation needle insertion angle; number of ablation needles etc.) may be altered. The process then continues to block 205 and another treatment plan 113 is generated based on the adjusted one or more ablation treatment contexts. This iterative process continues until the clinician validates a given treatment plan as acceptable.

Upon validating a given ablation treatment plan, informed at least in part via these teachings, the validated plan can then be administered to treat the corresponding patient with ablation and thereby address the patient's malady.

Further details that comport with these teachings will now be presented. It will be understood that the specific details of these examples are intended to serve an illustrative purpose and are not intended to suggest any particular limitations with respect to these teachings.

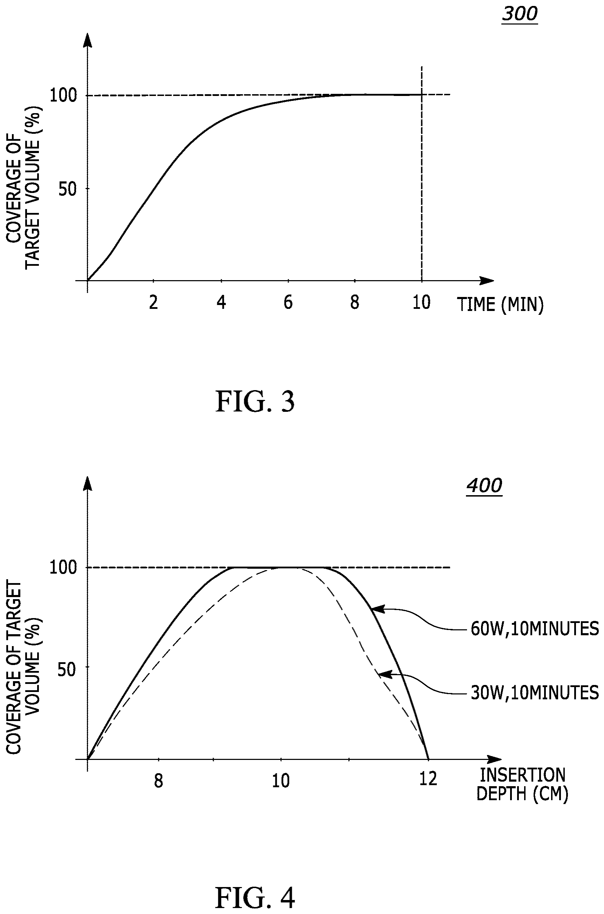

As one illustrative example in these regards, FIG. 3 presents a histogram 300 that presents target coverage by volume for a fixed power at different ablation durations.

During the treatment planning phase of an ablation procedure, there are numerous different factors that can affect the outcome, including both technical factors and physioanatomical factors. Examples of technical factors include, but are not limited to, needle type, the number of needles, needle insertion depth, ablation input power, ablation duration, and efficiency of the needle. Physioanatomical factors include, but are not limited to, variability of ablation zone dimensions (corresponding, for example, to tumor shape), heat transfer, a desired ablation margin, and a need to spare non-targeted tissue. Using a histogram 300 approach such as the one depicted in FIG. 3, the clinician or interventional oncologist can readily compare the results that correspond to different settings and conditions as regards the foregoing by changing the parameters corresponding to the above factors.

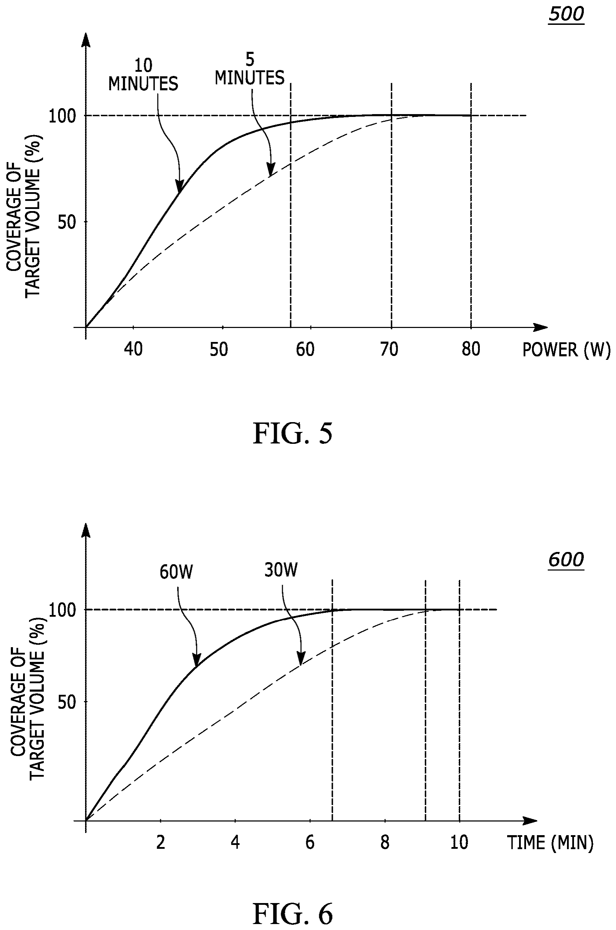

As another illustrative example in these regards, FIG. 4 presents an exemplary illustrative histogram 400 for different target coverage with regards to insertion depth of the needle for two different example ablation input power/time settings while FIG. 5 presents an exemplary illustrative histogram 500 of target coverage as a function of input power for two different ablation durations. FIG. 6, in turn, presents an exemplary illustrative histogram 600 of target coverage as a function of ablation duration for two different input powers.

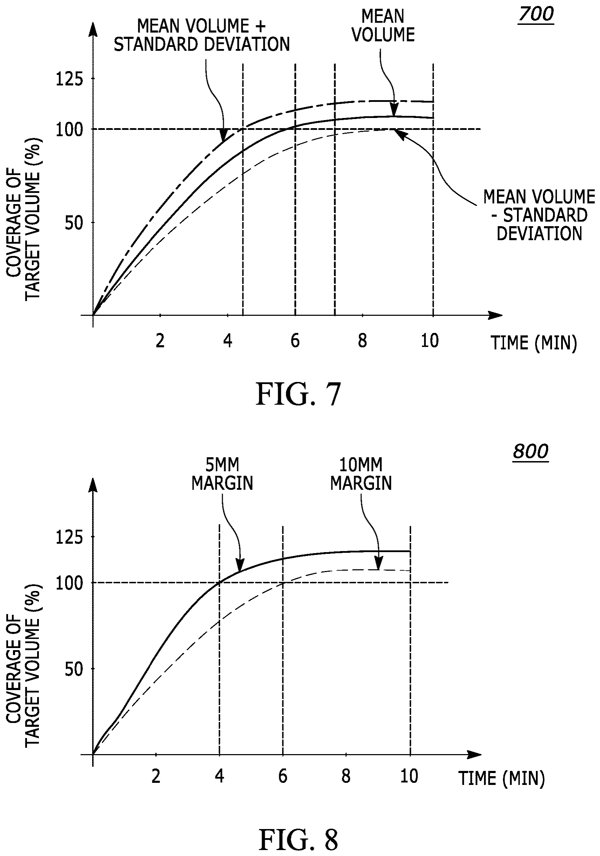

After finding an optimal insertion depth, input power, and ablation duration, these teachings can also serve to facilitate evaluation of ablation dimension variability as well as the desired margin for the selected insertion depth, input power, and ablation duration. FIG. 7 illustrates an exemplary illustrative histogram 700 for target coverage with regards to ablation zone dimension variability for a fixed input power.

As yet another illustrative example, FIG. 8 presents an exemplary illustrative histogram 800 of target coverage for a desired ablation margin for a fixed input power.

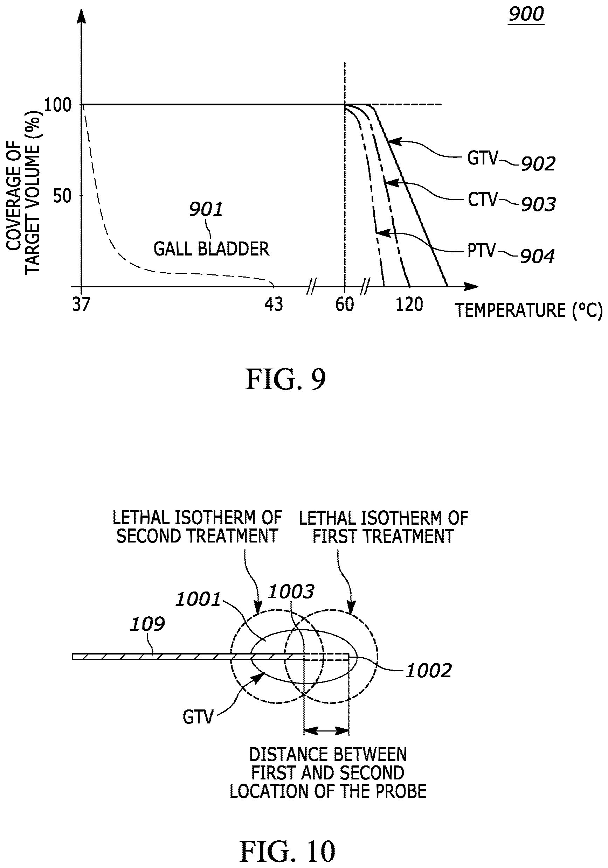

FIG. 9 presents an exemplary illustrative histogram 900 depicting target versus non-target coverage as a function of expected temperature for a fixed ablation setting. This example presumes a 60 °C lethal temperature and readily illustrates that the patient's entire gall bladder tissue 901 stays under 43 °C (and hence beneath the non-lethal temperature limit) while the gross tumor volume 902, clinical tumor volume 903, and planning tumor volume 904 regions are each and all completely covered by the ablation zone created by the selected ablation settings.

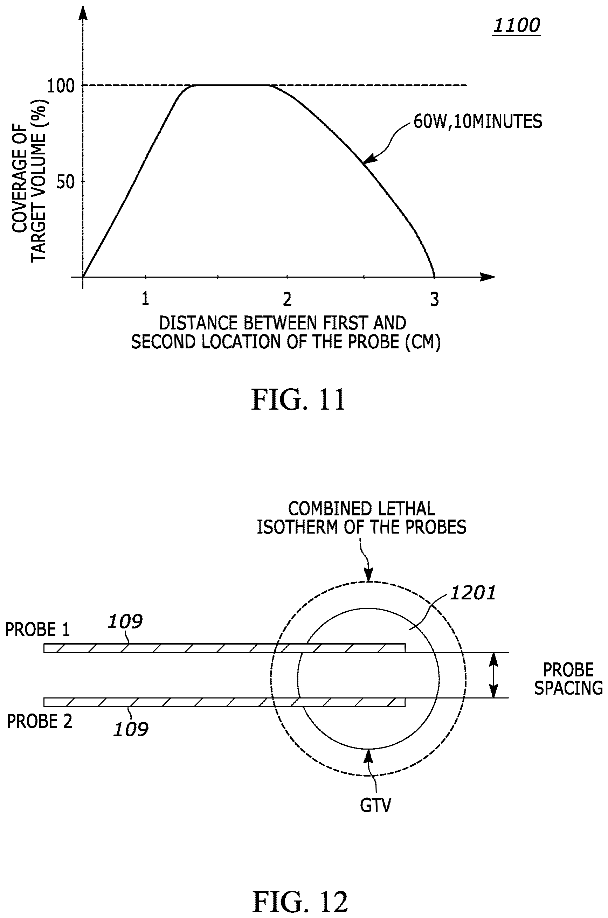

There are cases where the target volume has an irregular shape or has a size that is larger than the maximum area a single ablation needle can cover. FIG. 10 presents a simple illustrative example in such regards, where the target 1001 has an ellipsoidal shape. In such a case, the clinician might try to cover the entire volume 1001 by using repeated ablations. In particular, the ablation needle 109 is inserted into the target 1001 to a first location 1002, energized, repositioned to a second location 1003, and then energized for a second time. FIG. 11 presents an exemplary illustrative histogram 1100 for target coverage as a function of distance between the first location 1002 and the second location 1003 of the ablation probe 109 in such a treatment context. Histograms similar to those described above can also be generated per these teachings for such a situation.

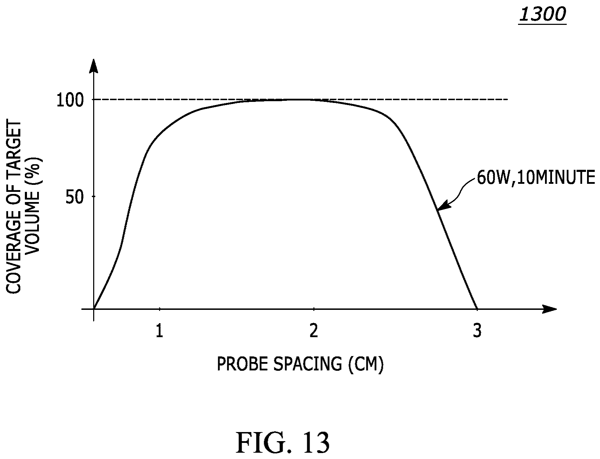

To accommodate the above-described situation where the target volume is too large to be covered by a single treatment by a single ablation needle, the clinician may instead prefer to utilize a plurality of ablation needles. FIG. 12 presents an illustrative example featuring a spherical shape target 1201 that the clinician seeks to treat and cover using two ablation needles 109 (denoted here as Probe 1 and Probe 2). FIG. 13 presents a corresponding exemplary illustrative histogram 1300 showing, two-dimensionally, target coverage as a function of distance between these two ablation needles 109. Two-dimensional histograms similar to those described above can again also be generated for such a case.

Further aspects of the invention are provided by the subject matter of the following clauses (where it will be understood that any of these clauses can be combined with any one of more of the other clauses as appropriate).

Clause 1. A method of generating a radiation treatment plan, comprising: by a control circuit: accessing three-dimensional image information of a target volume for a particular patient; accessing ablation zone information corresponding to a particular ablation treatment context; registering the three-dimensional image information of the target volume with the ablation zone information to generate resultant information representing the radiation treatment plan; generating a two-dimensional representation of target volume coverage by a corresponding ablation zone as a function of the resultant information; and presenting the two-dimensional representation of target volume coverage by the corresponding ablation zone via a user interface. According to one embodiment, the user interface is a computer display.

Clause 2. The method of clause 1, further comprising: following presenting the two-dimensional representation of target volume coverage by the corresponding ablation zone, determining when a user rejects the radiation treatment plan; and adjusting the ablation treatment context in response to the user rejection.

Clause3. The method of clause 1 or clause 2, wherein the three-dimensional image information of the target volume comprises three-dimensional computed tomography imagery.

Clause 4. The method of any one of clauses 1 to 3, wherein the ablation zone information comprises information based at least in part on an ablation zone dimension chart for a particular ablation needle.

Clause 5. The method of any one of clauses 1 to 4, wherein the ablation zone information comprises microwave ablation zone information.

Clause 6. The method of any one of clauses 1 to 5, wherein the two-dimensional representation of the target volume coverage by the corresponding ablation zone comprises a graph depicting a percentage of the target volume that is covered by the corresponding ablation zone.

Clause 7. The method of clause 6, wherein the graph further depicts a percentage of the target volume that is fully ablated by the corresponding ablation zone.

Clause8. The method of clause 7, wherein the graph further depicts a percentage of the target volume that is fully ablated by the corresponding ablation zone for each of a gross tumor volume, a clinical tumor volume, and a planning target volume.

Clause 9. The method of any one of clauses 1 to 8, wherein the particular ablation treatment context comprises a pre-treatment ablation treatment context.

Clause 10. The method of any one of clauses 1 to 9, wherein the particular ablation treatment context comprises a post-treatment ablation treatment context.

Clause 11. The method of any one of clauses 1 to 10, wherein the two-dimensional representation of the target volume coverage by the corresponding ablation zone is agnostic with respect to patient and ablation needle specifics to thereby allow for direct comparison with ablation results from other patients and/or ablation needles.

Clause12. An apparatus for generating a radiation treatment plan, comprising: a control circuit configured to: access three-dimensional image information of a target volume for a particular patient; access ablation zone information corresponding to a particular ablation treatment context; register the three-dimensional image information of the target volume with the ablation zone information to generate resultant information representing the radiation treatment plan; generate a two-dimensional representation of target volume coverage by a corresponding ablation zone as a function of the resultant information; and present the two-dimensional representation of target volume coverage by the corresponding ablation zone via a user interface. According to one embodiment, the user interface is a computer display.

Clause 13. The apparatus of clause 12, wherein the control circuit is further configured to: following presenting the two-dimensional representation of target volume coverage by the corresponding ablation zone, determine when a user rejects the radiation treatment plan; and in response to the user rejection, adjust the ablation treatment context.

Clause 14. The apparatus of clause12 or clause 13, wherein the three-dimensional image information of the target volume comprises three-dimensional computed tomography imagery.

Clause 15. The apparatus of any one of clauses 12 to 14, wherein the ablation zone information comprises information based at least in part on an ablation zone dimension chart for a particular ablation needle.

Clause 16. The apparatus of any one of clauses 12 to 15, wherein the ablation zone information comprises microwave ablation zone information.

Clause 17. The apparatus of any one of clauses 12 to 16, wherein the two-dimensional representation of the target volume coverage by the corresponding ablation zone comprises a graph depicting a percentage of the target volume that is covered by the corresponding ablation zone.

Clause 18. The apparatus of clause 17, wherein the graph further depicts a percentage of the target volume that is fully ablated by the corresponding ablation zone.

Clause 19. The apparatus of clause 18, wherein the graph further depicts a percentage of the target volume that is fully ablated by the corresponding ablation zone for each of a gross tumor volume, a clinical tumor volume, and a planning target volume.

Clause 20. The apparatus of any one of clauses 12 to 19, wherein the particular ablation treatment context comprises a pre-treatment ablation treatment context.

Clause 21. The apparatus of any one of clauses 12 to 20, wherein the particular ablation treatment context comprises a post-treatment ablation treatment context.

Clause 22. The apparatus of any one of clauses 12 to 21, wherein the two-dimensional representation of the target volume coverage by the corresponding ablation zone is agnostic with respect to patient and ablation needle specifics to thereby allow for direct comparison with ablation results from other patients and/or ablation needles.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method of generating a radiation treatment plan, comprising:
by a control circuit (101):
accessing (201) three-dimensional image information (202) of a target volume for a particular patient;
accessing (203) ablation zone information (204) corresponding to a particular ablation treatment context;
registering (205) the three-dimensional image information of the target volume with the ablation zone information to generate resultant information representing the radiation treatment plan;
generating (206) a two-dimensional representation of target volume coverage by a corresponding ablation zone as a function of the resultant information; and
presenting (207) the two-dimensional representation of target volume coverage by the corresponding ablation zone via a user interface.

2. The method of clause 1, further comprising:
following presenting the two-dimensional representation of target volume coverage by the corresponding ablation zone, determining when a user rejects the radiation treatment plan; and
adjusting the ablation treatment context in response to the user rejection.

3. The method of claim 1 or claim 2, wherein the three-dimensional image information (202) of the target volume comprises three-dimensional computed tomography imagery.

4. The method of any one of claims 1 to 3, wherein the ablation zone information (204) comprises:
information based at least in part on an ablation zone dimension chart for a particular ablation needle, and/or
microwave ablation zone information.

5. The method of any one of claims 1 to 4, wherein the two-dimensional representation of the target volume coverage by the corresponding ablation zone comprises a graph depicting a percentage of the target volume that is covered by the corresponding ablation zone.

6. The method of claim 5, wherein the graph further depicts a percentage of the target volume that is fully ablated by the corresponding ablation zone, and optionally depicts a percentage of the target volume that is fully ablated by the corresponding ablation zone for each of a gross tumor volume, a clinical tumor volume, and a planning target volume.

7. The method of any one of claims 1 to 6, wherein the particular ablation treatment context comprises a pre-treatment ablation treatment context and/or a post-treatment ablation treatment context.

8. The method of claim 7, wherein the two-dimensional representation of the target volume coverage by the corresponding ablation zone is agnostic with respect to patient and ablation needle specifics to thereby allow for direct comparison with ablation results from other patients and/or ablation needles.

9. An apparatus for generating a radiation treatment plan, comprising:
a control circuit configured to:
access (201) three-dimensional image information (202) of a target volume for a particular patient;
access (203) ablation zone information (204) corresponding to a particular ablation treatment context;
register (205) the three-dimensional image information (202) of the target volume with the ablation zone information (204) to generate resultant information representing the radiation treatment plan;
generate (206) a two-dimensional representation of target volume coverage by a corresponding ablation zone as a function of the resultant information; and
present (207) the two-dimensional representation of target volume coverage by the corresponding ablation zone via a user interface.

10. The apparatus of claim 9, wherein the control circuit is further configured to:
following presenting the two-dimensional representation of target volume coverage by the corresponding ablation zone, determine when a user rejects the radiation treatment plan; and
in response to the user rejection, adjust the ablation treatment context.

11. The apparatus of claim 9 or claim 10, wherein the three-dimensional image information (202) of the target volume comprises three-dimensional computed tomography imagery.

12. The apparatus of any one of claims 9 to 11, wherein the ablation zone information (204) comprises:
information based at least in part on an ablation zone dimension chart for a particular ablation needle; and/or
microwave ablation zone information.

13. The apparatus of any one of claims 9 to 11, wherein the two-dimensional representation of the target volume coverage by the corresponding ablation zone comprises a graph depicting a percentage of the target volume that is covered by the corresponding ablation zone, and optionally,
wherein the graph further depicts a percentage of the target volume that is fully ablated by the corresponding ablation zone, and optionally,
wherein the graph further depicts a percentage of the target volume that is fully ablated by the corresponding ablation zone for each of a gross tumor volume, a clinical tumor volume, and a planning target volume.

14. The apparatus of any one of claims 9 to 13, wherein the particular ablation treatment context comprises a pre-treatment ablation treatment context and/or a post-treatment ablation treatment context.

15. The apparatus of claim 14, wherein the two-dimensional representation of the target volume coverage by the corresponding ablation zone is agnostic with respect to patient and ablation needle specifics to thereby allow for direct comparison with ablation results from other patients and/or ablation needles.
